# FASCICULE DE BREVET EUROPEEN

(11) **EP 1 702 593 B1**
(45) Date de publication et mention de la délivrance du brevet: **09.12.2009**
(21) Numéro de dépôt: 06356030.4
(22) Date de dépôt: 17.03.2006
(51) Int. Cl.: A61F 5/00

(54) **Dispositif orthopédique modulable destiné à soutenir et/ou surélever une partie du pied**
Modulare orthopädische Vorrichtung, um einen Teil des Fußes zu stützen
Modular orthopedic device for supporting a part of the foot

(30) Priorité: 17.03.2005 FR 0502652
(43) Date de publication de la demande: 20.09.2006
(73) Titulaire: Gibaud, 42000 Saint Etienne (FR)
(72) Inventeur: Anquetil, Lionel, 03110 Serbannes (FR)
(74) Mandataire: Delorme, Nicolas

(56) Documents cités:
- DE-A1- 10 319 480
- DE-U1- 8 213 214
- DE-U1- 20 314 286
- GB-A- 651 477
- US-A- 5 732 481

## Description

La présente invention concerne un dispositif orthopédique modulable destiné à soutenir et/ou surélever une partie du pied.

Ce dispositif est destiné à être placé à l'intérieur de la chaussure d'un utilisateur sous la partie concernée du pied, par exemple sous la voûte plantaire ou sous le talon.

On connaît déjà de tels dispositifs qui sont formés d'une seule pièce. Ils présentent alors l'inconvénient de ne pas être modulables, c'est-à-dire adaptables à la hauteur de surélévation souhaitée. Cette hauteur peut varier d'une personne à l'autre, mais également pour une même personne selon les besoins. Par exemple, dans le cas d'une lésion du talon d'Achille, il est généralement souhaitable d'utiliser une talonnette d'épaisseur importante en début de traitement puis de diminuer progressivement l'épaisseur de la talonnette au fur et à mesure que l'utilisateur guérit et récupère la locomotion. Il est alors indispensable d'avoir recours à une série de talonnettes différentes, de hauteurs de plus en plus faibles.

On connaît également des dispositifs formés de plusieurs pièces élémentaires superposées pouvant être enlevées ou ajoutées en fonction des besoins.

On connaît par le document DE 103 19 480 de superposer plusieurs pièces élémentaires et de les relier par un système d'emboîture mâle/femelle.

Le document US 5 138 774 décrit une semelle dans laquelle est entre autres ménagé un logement destiné à recevoir une talonnette formée par la superposition de plusieurs pièces. Ces pièces ne sont pas assemblées les unes aux autres, mais simplement superposées, le logement assurant le maintien en position des pièces les unes par rapport aux autres. Il est donc nécessaire de prévoir une semelle pour recevoir les pièces superposées, ce qui n'est pas commode, ni même souhaitable dans certaines applications orthopédiques.

Le document EP O 254 662 décrit une semelle comportant notamment un dispositif de soutien de la voûte plantaire formé par la superposition de couches flexibles. Les couches sont assemblées les unes aux autres au moyen d'une matière adhésive permettant une association amovible, afin que l'on puisse enlever et remettre les couches de façon répétée. Toutefois, l'emploi d'une matière adhésive n'est pas optimal. En effet, avec ce mode d'assemblage, on ne peut pas enlever et remettre les couches indéfiniment, du fait de la perte du caractère adhésif avec le temps, à moins de remplacer la matière adhésive ou d'en ajouter, ce qui s'avère coûteux et peu adapté aux applications orthopédiques. En outre, la présence d'une matière adhésive rend les opérations de lavage ou de stérilisation délicates sinon impossibles. Or, ces opérations peuvent être nécessaires en orthopédie, notamment si le dispositif est destiné à être porté longtemps par une même personne ou s'il est souhaitable de pouvoir le réutiliser ultérieurement pour d'autres personnes.

Par ailleurs, les dispositifs connus n'offrent pas généralement une stabilisation satisfaisante du pied.

La présente invention vise à remédier aux inconvénients mentionnés ci-dessus, en fournissant un dispositif orthopédique qui soit modulable, c'est-à-dire qui puisse être adapté en fonction des besoins de l'utilisateur et notamment de sa guérison progressive, sans qu'il soit nécessaire d'envisager l'emploi d'un autre dispositif distinct ni de mettre en oeuvre des solutions peu commodes ou peu hygiéniques et qui offre une stabilisation du pied.

A cet effet, l'invention concerne un dispositif orthopédique modulable, destiné à être placé à l'intérieur d'une chaussure d'un utilisateur sous une partie du pied de ce dernier, afin de soutenir et/ou surélever ladite partie du pied, le dispositif comprenant au moins deux pièces élémentaires distinctes pouvant être assemblées l'une à l'autre en position superposée, de façon amovible, le dispositif pouvant évoluer selon les besoins de l'utilisateur par enlèvement d'une ou de plusieurs des pièces élémentaires assemblées. Selon une définition générale de l'invention, le dispositif est caractérisé en ce que :
- la face supérieure d'une première pièce et la face inférieure d'une deuxième pièce destinée à être assemblée sur la première pièce présentent des moyens réciproques d'emboîtement aptes à coopérer lorsque lesdites pièces sont superposées, de sorte que lesdites faces supérieure et inférieure soient sensiblement jointives ;
- la face inférieure de chacune des pièces susceptibles d'être en contact avec la semelle intérieure de la chaussure définit un plan général et est dépourvue d'élément faisant saillie par rapport au plan général de ladite face inférieure ;
- au moins l'une des pièces élémentaires présente une réservation qui autorise un écrasement local du dispositif lorsqu'il est disposé sous le pied de l'utilisateur.

L'assemblage des pièces élémentaires se faisant par simple emboîtement, on évite les inconvénients des dispositifs de l'art antérieur. Les moyens réciproques d'emboîtement permettent un positionnement approprié des pièces élémentaires les unes par rapport aux autres et un maintien en position pendant l'utilisation. A cet effet, les faces supérieure et inférieure des pièces assemblées sont sensiblement jointives sur une partie suffisante de leur surface pour assurer un appui et un maintien satisfaisants.

Par ailleurs, la présence de la réservation dans l'une des pièces superposées du dispositif autorise, lorsqu'une charge due au pied de l'utilisateur est appliquée à ce dispositif, un écrasement du dispositif dans lequel peut se loger le pied (plus spécialement le talon) qui contribue à stabiliser grandement le pied.

Pour l'adaptation du dispositif orthopédique aux besoins de l'utilisateur, on enlève une ou plusieurs pièces élémentaires, par exemple celle(s) située(s) en partie supérieure et/ou en partie inférieure du dispositif pour plus de praticité. Quels que soit le nombre et la position des pièces élémentaires enlevées, le dispositif orthopédique obtenu reste parfaitement ergonomique et stable puisque, d'une part, la pièce située en partie supérieure a toujours une forme adaptée à l'anatomie du pied et, d'autre part, la pièce située en partie inférieure repose toujours parfaitement sur la semelle de la chaussure et procure ainsi un bon appui. Cette dernière pièce peut comporter des orifices ou des saillies, pourvu que ceux-ci soient situés en deçà du plan général inférieur de la pièce.

Selon une réalisation possible, la face supérieure de la première pièce et la face inférieure de la deuxième pièce définissent un plan général et les moyens réciproques d'emboîtement sont aptes à bloquer la translation de la première pièce par rapport à la deuxième pièce dans toutes les directions parallèles audit plan général.

Les moyens réciproques d'emboîtement comprennent par exemple au moins un premier élément en saillie et/ou en creux ménagé sur la face supérieure de la première pièce et au moins un deuxième élément en saillie et/ou en creux ménagé sur la face inférieure de la deuxième pièce. Selon plusieurs réalisations possibles :
- le premier élément, respectivement le deuxième élément, présente la forme d'une cavité arrondie, le deuxième élément, respectivement le premier élément, présentant la forme d'un dôme ;
- le premier élément, respectivement le deuxième élément, présente la forme d'un cylindre en saillie, le deuxième élément, respectivement le premier élément, présentant la forme d'un orifice borgne ou traversant de forme intérieure cylindrique ;
- le premier élément, respectivement le deuxième élément, présente la forme d'une couronne en saillie, le deuxième élément, respectivement le premier élément, présentant la forme d'une rainure en couronne.

Les pièces élémentaires présentent par exemple des épaisseurs sensiblement identiques.

Par ailleurs, ces pièces peuvent être réalisées en un matériau élastiquement déformable appartenant au groupe formé par les gommes, les élastomères, les élastomères de silicone. Elles peuvent ainsi s'adapter parfaitement à la forme de la partie du pied concernée. Le caractère non glissant du matériau peut être une propriété recherchée. Enfin, ces matériaux permettent de laver et stériliser les pièces élémentaires. Ils peuvent également faire l'objet d'un traitement anti bactérien.

Selon une réalisation possible, lorsque les pièces élémentaires sont assemblées les unes aux autres, leurs faces latérales forment une surface sensiblement continue sans marches. On a ainsi l'impression que le dispositif est formé d'une seule pièce. Cette caractéristique est avantageuse en termes d'esthétique, mais rend également plus aisée la mise en place du dispositif dans la chaussure.

Le dispositif constitue, par exemple, une talonnette.

Dans cette application, la réservation est avantageusement localisée sous le talon pour permettre un écrasement du dispositif dans lequel peut se loger le talon qui constitue le point d'appui maximum de l'arrière du pied.

Dans ce cas, les moyens réciproques d'emboîtement sont de préférence positionnés de sorte à être localisés sous le talon, c'est-à-dire dans la zone où les sollicitations sont maximales et pourraient conduire au déplacement des pièces élémentaires les unes par rapport aux autres. Les pièces élémentaires peuvent présenter une face inférieure définissant un plan inférieur général et une face supérieure définissant un plan supérieur général incliné par rapport au plan inférieur général d'un angle compris entre 4 et 15°, voire entre 5 et 10°, voire encore entre 6 et 8°.

Pour sa bonne compréhension, l'invention est à nouveau décrite ci-dessous en référence aux figures annexées représentant, à titre d'exemples non limitatifs, plusieurs formes de réalisation possibles du dispositif orthopédique.
La figure 1 est une vue latérale du pied d'un utilisateur sous le talon duquel est placée une talonnette selon l'invention ;
La figure 2 est une vue de dessus du pied et de la talonnette de la figure 1 ;
La figure 3 est une vue agrandie, en coupe longitudinale médiane, de la talonnette de la figure 1, selon un premier mode de réalisation, seule la pièce intermédiaire étant hachurée pour faciliter la compréhension ;
La figure 4 est une vue en perspective éclatée de la talonnette de la figure 3.

Une talonnette 1, destinée à être placée sous le talon du pied 2 d'un utilisateur à l'intérieur d'une chaussure (non représentée), comprend trois pièces superposées et emboîtées l'une dans l'autre, à savoir une pièce de base 3 en partie inférieure, une pièce intermédiaire 4 et une pièce permanente 5 en partie supérieure.

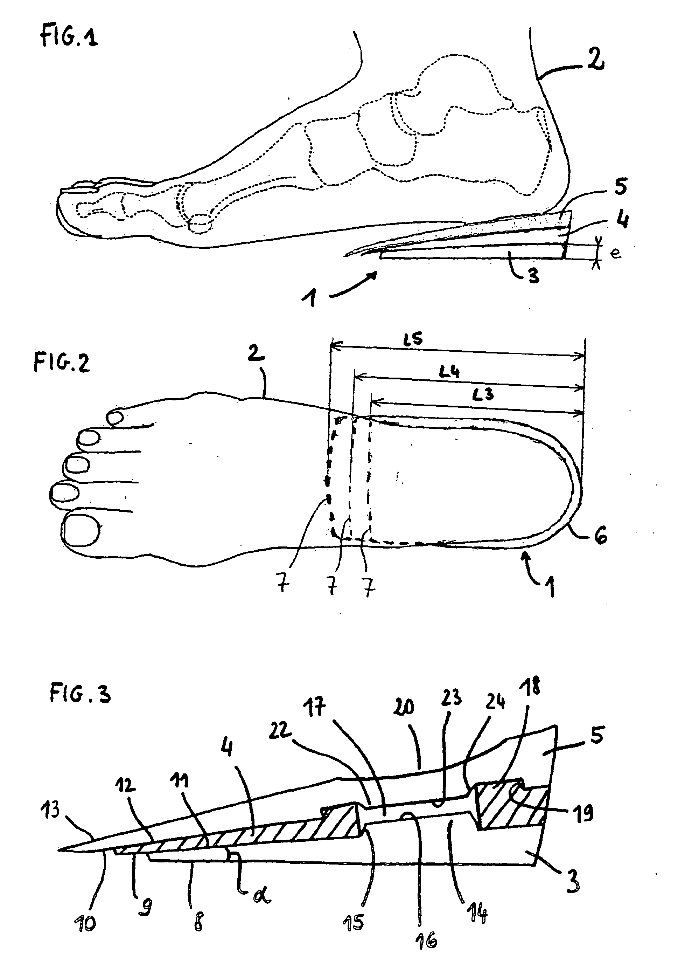

Chacune des pièces 3, 4, 5 présente des faces latérales et arrière définissant un contour 6 en U et une face avant 7 sensiblement droite et perpendiculaire aux faces latérales. Les longueurs L3, L4, L5 des pièces 3, 4, 5 entre la face arrière et la face avant 7 sont différentes et croissantes dans cet ordre. A titre d'exemple, les longueurs L3, L4, L5 peuvent être de l'ordre de 8, 9 et 10 cm respectivement. En outre, la face arrière de chacune des pièces 3, 4, 5 est légèrement inclinée du bas vers le haut et de l'avant vers l'arrière, comme illustré sur la figure 3.

Par ailleurs, chacune des pièces 3, 4, 5 présente une face inférieure, respectivement 8, 9 et 10, définissant un plan inférieur général et une face supérieure, respectivement 11, 12 et 13, définissant un plan supérieur général. La face supérieure d'une pièce donnée est inclinée par rapport à la face inférieure de la même pièce d'un angle α de l'ordre de 6 à 8°. Ainsi, l'épaisseur des pièces 3, 4, 5 entre la face inférieure et la face supérieure diminue de l'arrière vers l'avant du pied. A titre d'exemple, l'épaisseur e des pièces 3, 4, 5 est identique au niveau de la face arrière et de l'ordre de 0,6 à 1 cm, par exemple de 0,7 cm. La diminution de l'épaisseur en allant vers la face avant 7 se fait de façon sensiblement continue et identique pour les pièces 3, 4, 5.

Lorsque les pièces 3, 4, 5 sont assemblées les unes aux autres, leurs faces latérales et arrières forment une surface sensiblement continue, la talonnette 1 ne présentant donc pas de marches à sa périphérie. La largeur de la talonnette 1 est suffisante pour permettre au pied 2 de venir en appui sur elle, la talonnette 1 dépassant de préférence légèrement de chaque côté du talon. De plus, la longueur L5 de la pièce permanente 5 est suffisante pour que la talonnette 1 s'étende depuis le talon jusqu'au milieu du pied environ.

On décrit à présent plus précisément les moyens réciproques d'emboîtement des pièces 3, 4, 5.

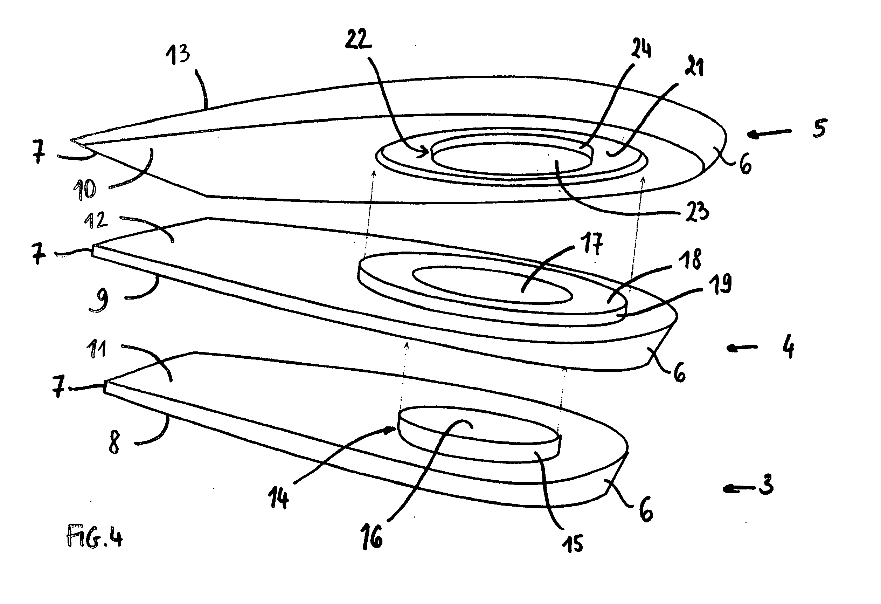

On se réfère tout d'abord aux figures 3 et 4, qui illustrent un premier mode de réalisation.

La face inférieure 8 de la pièce de base 3 est plane, et destinée à être placée contre la semelle intérieure de la chaussure. La face supérieure 11 comprend une majeure partie plane et est pourvue d'un premier élément 14 cylindrique faisant saillie sensiblement perpendiculairement de ladite partie plane. Le premier élément 14 est situé en partie arrière et sensiblement au milieu - transversalement - de la face supérieure 11 de la pièce de base 3, de sorte à être situé au droit du talon lorsque le pied de l'utilisateur repose sur la talonnette 1. Le premier élément 14 présente une hauteur inférieure à l'épaisseur de la pièce intermédiaire 4 dans la portion de ladite pièce intermédiaire 4 destinée à être en regard dudit premier élément 14. De plus, la paroi latérale 15 du premier élément 14 est légèrement convergente en tronc de cône depuis sa base vers son extrémité libre 16. Par exemple, le premier élément 14 présente un diamètre de l'ordre de 2 à 3 cm et une hauteur de l'ordre de 2 à 5 mm.

La face inférieure 9 de la pièce intermédiaire 4 est plane, et destinée à être placée contre la face supérieure 11 de la pièce de base 3 lorsque la talonnette 1 est formée des trois pièces 3, 4, 5. La pièce intermédiaire 4 comprend une réservation 17 traversante, dont le diamètre est sensiblement égal au diamètre du premier élément cylindrique 14 au niveau de sa base, le premier élément cylindrique 14 étant destiné à être engagé dans la réservation 17. La face supérieure 12 de la pièce intermédiaire 4 comprend une majeure partie plane et une couronne 18 faisant saillie sensiblement perpendiculairement de ladite partie plane autour de la réservation 17, le diamètre intérieur de la couronne 18 étant sensiblement identique au diamètre de la réservation 17. Le diamètre extérieur de la couronne 18 est par exemple de l'ordre de 3 à 5 cm et l'épaisseur de l'ordre de 2 à 5 mm. De plus, la face latérale extérieure 19 de la couronne 18 converge légèrement en tronc de cône du bas vers le haut.

La face supérieure 13 de la pièce permanente 5 est plane et destinée à être placée directement sous le talon. En variante, la face supérieure 13 comprend une cavité arrondie 20 dans laquelle le talon est destiné à venir se loger pour un meilleur positionnement du pied par rapport à la talonnette 1. Dans ce cas, la cavité 20 est située dans l'alignement du premier élément cylindrique 14.

La face inférieure 10 de la pièce permanente 5 est destinée à être placée contre la face supérieure 12 de la pièce intermédiaire 4 lorsque la talonnette 1 est formée des trois pièces 3, 4, 5. La face inférieure 10 comprend une majeure partie plane et une rainure 21 annulaire dont le diamètre intérieur et le diamètre extérieur sont sensiblement identiques respectivement au diamètre intérieur et au diamètre extérieur, à sa base, de la couronne 18 en saillie de la pièce intermédiaire 4. La rainure 21 définit en son centre un deuxième élément cylindrique 22 faisant saillie vers le bas, sensiblement perpendiculairement à la partie plane de la face inférieure 10. La hauteur du deuxième élément 22 est telle que l'extrémité inférieure libre 23 du deuxième élément 22 est située sensiblement dans le plan général défini par la face inférieure 10. De plus, la paroi latérale 24 du deuxième élément 22 est légèrement convergente en tronc de cône depuis sa base vers son extrémité libre 23. La rainure 21 et le deuxième élément cylindrique 22 sont destinés à coopérer respectivement avec la couronne 18 et la réservation 17 de la pièce intermédiaire 4.

L'assemblage des trois pièces 3, 4, 5 pour constituer la talonnette 1 s'effectue de préférence du bas vers le haut : la pièce de base 3 est placée dans la chaussure de l'utilisateur, puis la pièce intermédiaire 4 est superposée à la pièce de base 3 et assemblée à celle-ci par engagement du premier élément cylindrique 14 dans la réservation 17, cet engagement étant facilité par la forme en tronc de cône du premier élément cylindrique 14. Enfin, la pièce permanente 5 est superposée à la pièce intermédiaire 4 et assemblée à celle-ci par engagement du deuxième élément cylindrique 22 dans la réservation 17 et de la couronne 18 dans la rainure 21, cet engagement étant facilité par la forme en tronc de cône de la face latérale extérieure 19 de la couronne 18 d'une part et de la paroi latérale 24 du deuxième élément 22 d'autre part.

Dans cette position (voir figure 3), les extrémités libres 16 et 23 des premier et deuxième éléments cylindriques 14, 22 ne sont pas jointives, ce qui permet une déformation de la talonnette 1 par écrasement sous l'effet du poids de l'utilisateur, la talonnette 1 s'adaptant ainsi parfaitement à l'anatomie du pied 2.

Dans l'exemple représenté, la réservation 17 a ainsi une double fonction puisqu'elle permet de réaliser l'emboîtement des pièces permanentes 2, 4, 5 et permet également de créer une zone susceptible de se déformer. Cette zone se trouve au droit du talon et permet ainsi de stabiliser efficacement le pied du patient. Il doit être précisé qu'il pourrait tout à fait être envisagé de dissocier la réservation des moyens d'emboîtement. Il pourrait ainsi être envisagé, pur la pièce intermédiaire, une réservation et distinctes de celle-ci sur sa face supérieure 12, une couronne qui pourrait s'emboîter dans une rainure annulaire prévue dans la pièce permanence et, sur sa face inférieure 9, un épaulement annulaire dans lequel pourrait s'emboîter l'élément cylindrique 14.

La réservation peut également être ménagée dans la pièce permanente 5 ; dans ce cas la réservation sera de préférence borgne sur sa face supérieure contre laquelle le pied de l'utilisateur viendra en appui.

En fonction des besoins de l'utilisateur, il est possible de diminuer l'épaisseur de la talonnette 1 par enlèvement d'une ou de plusieurs des pièces élémentaires qui la constituent. Cet enlèvement s'effectue très simplement par traction, et se fait ici par la partie inférieure de la talonnette 1 : il est donc nécessaire, au préalable, de sortir la talonnette 1 de la chaussure.

Dans un premier temps, on peut n'enlever que la pièce de base 3. C'est alors la pièce intermédiaire 4 qui se trouve au contact de la semelle intérieure de la chaussure. La face inférieure 9 de la pièce intermédiaire 4 étant plane, la talonnette 1 conserve un bon appui dans la chaussure.

Si nécessaire, on peut ensuite enlever la pièce intermédiaire 4, la talonnette 1 n'étant alors constituée que de la pièce permanente 5, qui repose directement sur la semelle intérieure de la chaussure. Là encore, l'appui est excellent car la face inférieure 10 de la pièce permanente 5 définit un plan général et l'extrémité libre 23 du deuxième élément cylindrique est située sensiblement dans ce plan.

Il va de soi que l'invention n'est pas limitée aux formes de réalisation décrites ci-dessus à titre d'exemples mais qu'elle en embrasse au contraire toutes les variantes de réalisation.

Ainsi, la talonnette pourrait comprendre un nombre différent de pièces élémentaires (2, 4 ou davantage, pourvu qu'il soit toujours possible de ménager dans les pièces élémentaires des moyens d'emboîtement complémentaire et qu'il soit toujours possible de ménager une réservation dans au moins l'une des pièces élémentaires, de sorte à permettre une déformation locale par exemple au droit du talon pour stabiliser le pied).

Par ailleurs, les moyens d'emboîtement complémentaires pourraient présenter des formes différentes.

Par exemple, une pièce élémentaire pourrait comporter des saillies s'étendant vers le bas et définissant entre elles des rainures, tandis que la pièce élémentaire destinée à lui être accolée en partie inférieure comporterait des saillies s'étendant vers le haut et positionnées en regard des rainures. Les saillies pourraient être rectilignes, d'orientation longitudinale, transversale ou oblique. Le non glissement dans la direction des saillies pourrait être assuré par l'emploi d'un matériau adapté pour la réalisation de la talonnette.

## Revendications

1. Dispositif orthopédique modulable, destiné à être placé à l'intérieur d'une chaussure d'un utilisateur sous une partie du pied (2) de ce dernier, afin de soutenir et/ou surélever ladite partie du pied, le dispositif (1) comprenant au moins deux pièces élémentaires (3, 4, 5) distinctes pouvant être assemblées l'une à l'autre en position superposée, de façon amovible, le dispositif pouvant évoluer selon les besoins de l'utilisateur par enlèvement d'une ou de plusieurs des pièces élémentaires assemblées,
- la face supérieure (11, 12, 13) d'une première pièce et la face inférieure (8, 9, 10) d'une deuxième pièce destinée à être assemblée sur la première pièce présentent des moyens réciproques d'emboîtement (14, 17, 18, 22, 25, 26, 27, 28) aptes à coopérer lorsque lesdites pièces sont superposées, de sorte que lesdites faces supérieure et inférieure soient sensiblement jointives ;
- la face inférieure (8, 9, 10) de chacune des pièces (3, 4, 5) susceptibles d'être en contact avec la semelle intérieure de la chaussure définit un plan général et est dépourvue d'élément faisant saillie par rapport au plan général de ladite face inférieure (8, 9, 10) ;
- la face supérieure (11, 12, 13) de chacune des pièces (3, 4, 5) susceptibles d'être en contact avec la partie du pied (2) de l'utilisateur présente une forme adaptée à la forme de ladite partie du pied **caractérisé en ce que**
- au moins l'une des pièces élémentaires présente une réservation traversante (17) pratiquée dans l'épaisseur de ladite pièce élémentaire qui autorise un écrasement local du dispositif lorsqu'il est disposé sous le pied de l'utilisateur.

2. Dispositif selon la revendication 1, **caractérisé en ce que** la face supérieure (11, 12, 13) de la première pièce et la face inférieure (8, 9, 10) de la deuxième pièce définissent un plan général et **en ce que** les moyens réciproques d'emboîtement sont aptes à bloquer la translation de la première pièce (3, 4) par rapport à la deuxième pièce (4, 5) dans toutes les directions parallèles audit plan général.

3. Dispositif selon la revendication 1 ou 2, **caractérisé en ce que** les moyens réciproques d'emboîtement comprennent au moins un premier élément en saillie et/ou en creux ménagé sur la face supérieure (11, 12, 13) de la première pièce (3, 4) et au moins un deuxième élément en saillie et/ou en creux ménagé sur la face inférieure (8, 9, 10) de la deuxième pièce (4, 5).

4. Dispositif selon la revendication 3, **caractérisé en ce que** le premier élément, respectivement le deuxième élément, présente la forme d'une cavité arrondie (25, 27), le deuxième élément, respectivement le premier élément, présentant la forme d'un dôme (26, 28).

5. Dispositif selon la revendication 3, **caractérisé en ce que** le premier élément, respectivement le deuxième élément, présente la forme d'un cylindre en saillie (14, 22), le deuxième élément, respectivement le premier élément, présentant la forme d'une réservation (17) borgne ou traversant de forme intérieure cylindrique.

6. Dispositif selon la revendication 3, **caractérisé en ce que** le premier élément, respectivement le deuxième élément, présente la forme d'une couronne en saillie (18), le deuxième élément, respectivement le premier élément, présentant la forme d'une rainure (21) annulaire.

7. Dispositif selon l'une des revendications 1 à 6, **caractérisé en ce que** les pièces élémentaires (3, 4, 5) présentent des épaisseurs sensiblement identiques.

8. Dispositif selon l'une des revendications 1 à 7, **caractérisé en ce que** les pièces élémentaires (3, 4, 5) sont réalisées en un matériau élastiquement déformable appartenant au groupe formé par les gommes, les élastomères, les élastomères de silicone.

9. Dispositif selon l'une des revendications 1 à 8, **caractérisé en ce que**, lorsque les pièces élémentaires (3, 4, 5) sont assemblées les unes aux autres, leurs faces latérales forment une surface sensiblement continue sans marches.

10. Dispositif selon l'une des revendications 1 à 9, **caractérisé en ce qu'**il constitue une talonnette (1), les pièces élémentaires (3, 4, 5) présentant chacune un contour (6) en U en vue de dessus et une épaisseur diminuant de l'arrière vers l'avant du pied.

11. Dispositif selon la revendication 10, **caractérisé en ce que** la réservation (17) est positionnée de sorte à être localisés sous le talon.

12. Dispositif selon la revendication 10 ou la revendication 11, **caractérisé en ce que** les moyens réciproques d'emboîtement sont positionnés de sorte à être localisés sous le talon.

13. Dispositif selon l'une des revendications 10 ou 12, **caractérisé en ce que** les pièces élémentaires (3, 4, 5) présentent une face inférieure (8, 9, 10) définissant un plan inférieur général et une face supérieure (11, 12, 13) définissant un plan supérieur général incliné par rapport au plan inférieur général d'un angle compris entre 4 et 15°.

14. Dispositif selon l'une des revendications 10 à 13, **caractérisé en ce que** la talonnette (1) comprend trois pièces élémentaires, à savoir une pièce permanente (5) destinée à être au contact du talon, une pièce intermédiaire (4) destinée à être assemblée sous la pièce permanente (5), et une pièce de base (3) destinée à être assemblée sous la pièce intermédiaire (4), l'évolution de la talonnette (1) s'effectuant par enlèvement des pièces élémentaires (3, 4, 5) depuis la partie inférieure de la talonnette (1).

15. Dispositif selon les revendications 13 et 14, **caractérisé en ce que** :
- la pièce de base (3) comprend un premier élément cylindrique (14) faisant saillie sensiblement perpendiculairement de la face supérieure (11) et présentant une hauteur inférieure à l'épaisseur de la pièce intermédiaire (4) dans la portion de ladite pièce intermédiaire (4) destinée à être en regard dudit premier élément (14) ;
- la pièce intermédiaire (4) comprend une réservation (17) traversant de diamètre sensiblement égal au diamètre du premier élément cylindrique (14), le premier élément cylindrique (14) étant destiné à être engagé dans la réservation (17), et une couronne (18) faisant saillie sensiblement perpendiculairement de la face supérieure (12) autour de la réservation (17), le diamètre intérieur de la couronne (18) étant sensiblement identique au diamètre de la réservation (17) ;
- la pièce permanente (5) comprend une rainure (21) en couronne pratiquée depuis la face inférieure (10), la rainure (21) présentant un diamètre intérieur et un diamètre extérieur sensiblement identiques respectivement au diamètre intérieur et au diamètre extérieur de la couronne (18) en saillie de la pièce intermédiaire (4), la rainure (21) définissant en son centre un deuxième élément cylindrique (22) en saillie dont l'extrémité inférieure (23) est située sensiblement dans le plan inférieur général de la pièce permanente (5), la rainure (21) et le deuxième élément cylindrique (22) étant destinés à coopérer respectivement avec la couronne (18) et la réservation (17).

## Claims

1. A modular orthopedic device to be placed inside a shoe of a user under part of the foot (2) of said user, in order to support and/or raise said part of the foot, the device (1) comprising at least two distinct elementary pieces (3, 4, 5) which can be assembled to each other in a superimposed position, removably, the device being able to evolve according to the needs of the user through removal of one or several of the assembled elementary pieces,
- the upper face (11, 12, 13) of a first piece and the lower face (8, 9, 10) of a second piece intended to be assembled on the first piece having mutual snapping means (14, 17, 18, 22, 25, 26, 27, 28) capable of cooperating when said pieces are superimposed, such that said upper and lower faces are essentially contiguous;
- the lower face (8, 9, 10) of each of the pieces (3, 4, 5) capable of being in contact with the inner sole of the shoe defines a general plane and does not have an element protruding in relation to the general place of said lower face (8, 9, 10);
- the upper face (11, 12, 13) of each of the pieces (3, 4, 5) capable of being in contact with the part of the foot (2) of the user has a shape adapted to the shape of said part of the foot, **characterized in that**
- at least one of the elementary pieces has a through reservation (17) formed in the thickness of said elementary piece which allows local crushing of the device when it is disposed under the foot of the user.

2. The device according to claim 1, **characterized in that** the upper face (11, 12, 13) of the first piece and the lower face (8, 9, 10) of the second piece define a general plane, and **in that** the mutual snapping means are capable of blocking the translation of the first piece (3, 4) in relation to the second piece (4, 5) in all directions parallel to said general plane.

3. The device according to claim 1 or 2, **characterized in that** the mutual snapping means comprise at least one first element protruding and/or in a hollow arranged on the upper face (11, 12, 13) of the first piece (3, 4) and at least one second element protruding and/or in a hollow arranged on the lower face (8, 9, 10) of the second piece (4, 5).

4. The device according to claim 3, **characterized in that** the first element, second element, respectively, is in the form of a rounded cavity (25, 27), the second element, first element, respectively, being in the form of a dome (26, 28).

5. The device according to claim 3, **characterized in that** the first element, second element, respectively, is in the form of a protruding cylinder (14, 22), the second element, first element, respectively, being in the form of a blind or through reservation (17) with a cylindrical inner shape.

6. The device according to claim 3, **characterized in that** the first element, second element, respectively, is in the form of a protruding crown (18), the second element, first element, respectively, being in the form of an annular groove (21).

7. The device according to one of claims 1 to 6, **characterized in that** the elementary pieces (3, 4, 5) have essentially identical thicknesses.

8. The device according to one of claims 1 to 7, **characterized in that** the elementary pieces (3, 4, 5) are made in an elastically deformable material belonging to the group made up of rubbers, elastomers, silicone elastomers.

9. The device according to one of claims 1 to 8, **characterized in that**, when the elementary pieces (3, 4, 5) are assembled to each other, their lateral faces form an essentially continuous surface without steps.

10. The device according to one of claims 1 to 9, **characterized in that** it constitutes a heel-pad (1), the elementary pieces (3, 4, 5) each having a contour (6) which is U-shaped as seen from above and a thickness which decreases from the rear toward the front of the foot.

11. The device according to claim 10, **characterized in that** the reservation (17) is positioned so as to be located under the heel.

12. The device according to claim 10 or claim 11, **characterized in that** the mutual snapping means are positioned so as to be located under the heel.

13. The device according to one of claims 10 or 12, **characterized in that** the elementary pieces (3, 4, 5) have a lower face (8, 9, 10) defining a general lower plane and an upper face (11,12,13) defining a general upper plane inclined in relation to the lower general plane by an angle between 4 and 15°.

14. The device according to one of claims 10 to 13, **characterized in that** the heel-pad (1) comprises three elementary pieces, namely a permanent piece (5) intended to be in contact with the heel, an intermediate piece (4) intended to be assembled under the permanent piece (5), and a base piece (3) intended to be assembled under the intermediate piece (4), the evolution of the heel-pad (1) being done through removal of the elementary pieces (3, 4, 5) from the lower part of the heel-pad (1).

15. The device according to claims 13 and 14, **characterized in that**:
- the base piece (3) comprises a first cylindrical element (14) protruding essentially perpendicular to the upper face (11) and having a height smaller than the thickness of the intermediate piece (4) in the portion of said intermediate piece (4) intended to be opposite said first element (14);
- the intermediate piece (4) comprises a through reservation (17) with a diameter essentially equal to the diameter of the first cylindrical element (14), the first cylindrical element (14) being intended to be engaged in the reservation (17), and a crown (18) protruding essentially perpendicular to the upper face (12) around the reservation (17), the inner diameter of the crown (18) being essentially identical to the diameter of the reservation (17);
- the permanent piece (5) comprises a crown groove (21) formed from the lower face (10), the groove (21) having an inner diameter and an outer diameter essentially identical to the inner diameter and the outer diameter, respectively, of the crown (18) protruding from the intermediate piece (4), the groove (21) defining, in its center, a second protruding cylindrical element (22) whereof the lower end (23) is situated essentially in the lower general plane of the permanent piece (5), the groove (21) and the second cylindrical element (22) being intended to cooperate with the crown (18) and the reservation (17), respectively.

## Patentansprüche

1. Modulare orthopädische Vorrichtung, die dazu bestimmt ist, in dem Schuh eines Benutzers unter einem Teilstück des Fußes (2) desselben platziert zu werden, um das Teilstück des Fußes zu stützen und/oder anzuheben, wobei die Vorrichtung (1) mindestens zwei gesonderte elementare Teile (3, 4, 5) umfasst, die in übereinanderliegender Stellung lösbar miteinander verbindbar sind, wobei die Vorrichtung nach Bedarf des Benutzers durch Entfernen eines oder mehrerer der verbundenen elementaren Teile weiterentwickelbar ist,
- die Oberseite (11, 12, 13) eines ersten Teils und die Unterseite (8, 9, 10) eines zweiten Teils, das dazu bestimmt ist, mit dem zweiten Teil verbunden zu werden, reziproke Verschachtelungsmittel (14, 17, 18, 22, 25, 2B, 27, 28) aufweisen, die imstande sind zusammenzuarbeiten, wenn die Teile übereinander liegen, so dass die Ober- und Unterseite etwa aneinander stoßen,
- die Unterseite (8, 9, 10) jedes der Teile (3, 4, 5), die befähigt sind, mit der Innensohle des Schuhs im Kontakt zu sein, eine allgemeine Ebene definiert und mit einem Element versehen ist, das im Verhältnis zur allgemeinen Ebene der Unterseite (8, 9, 10) hervorsteht,
- die Oberseite (11, 12, 13) jedes der Teile (3, 4, 5), die befähigt sind, mit dem Abschnitt des Fußes (2) des Benutzers im Kontakt zu sein, eine Form aufweist, die an die Form des Fußteilstücks angepasst ist, **dadurch gekennzeichnet, dass**
- mindestens eines der elementaren Teile eine durchgehende, in die Dicke des elementaren Teils eingebrachte Aussparung (17) aufweist, die eine lokale Stauchung der Vorrichtung erlaubt, wenn sie unter dem Fuß des Benutzers angeordnet ist.

2. Vorrichtung nach Anspruch 1, **dadurch gekennzeichnet, dass** die Oberseite (11, 12, 13) des ersten Teils und die Unterseite (8, 9, 10) eines zweiten Teils eine allgemeine Ebene definieren und dadurch, dass die reziproken Verschachtelungsmittel imstande sind, die Verschiebung des ersten Teils (3, 4) im Verhältnis zum zweiten Teil (4, 5) in alle Richtungen parallel zur allgemeinen Ebene zu blockieren.

3. Vorrichtung nach Anspruch 1 oder 2, **dadurch gekennzeichnet, dass** die reziproken Verschachtelungsmittel mindestens ein erstes hervorstehendes und/oder hohles Element umfassen, das auf der Oberseite (11, 12, 13) des ersten Teils (3, 4) angeordnet ist und mindestens ein zweites hervorstehendes und/oder hohles Element, das auf der Unterseite (8, 9, 10) des zweiten Teils (4, 5) angeordnet ist.

4. Vorrichtung nach Anspruch 3, **dadurch gekennzeichnet, dass** das erste Element beziehungsweise das zweite Element die Form einer abgerundeten Vertiefung (25, 27) aufweist, wobei das zweite Element beziehungsweise das erste Element die Form einer Kuppel (26, 28) aufweist.

5. Vorrichtung nach Anspruch 3, **dadurch gekennzeichnet, dass** das erste Element beziehungsweise das zweite Element die Form eines vorspringenden Zylinders (14, 22) aufweist, wobei das zweite Element beziehungsweise das erste Element die Form einer blinden oder durchgehenden Aussparung (17) mit einer inneren zylindrischen Form aufweist.

6. Vorrichtung nach Anspruch 3, **dadurch gekennzeichnet, dass** das erste Element beziehungsweise das zweite Element die Form eines vorspringenden Kranzes (18) aufweist, wobei das zweite Element beziehungsweise das erste Element die Form einer ringförmigen Nut (21) aufweist.

7. Vorrichtung nach einem der Ansprüche 1 bis 6, **dadurch gekennzeichnet, dass** die elementaren Teile (3, 4, 5) etwa gleich dick sind.

8. Vorrichtung nach einem der Ansprüche 1 bis 7, **dadurch gekennzeichnet, dass** die elementaren Teile (3, 4, 5) aus einem elastisch deformierbaren Material gefertigt sind, das zu der Gruppe gehört, die von den Gummis, den Elastomeren, den Silikonelastomeren gebildet wird.

9. Vorrichtung nach einem der Ansprüche 1 bis 8, **dadurch gekennzeichnet, dass**, wenn die elementaren Teile (3, 4, 5) miteinander verbunden sind, ihre seitlichen Seiten eine etwa kontinuierliche Fläche ohne Stufen bilden.

10. Vorrichtung nach einem der Ansprüche 1 bis 9, **dadurch gekennzeichnet, dass** sie einen Keilabsatz (1) bildet, wobei die elementaren Teile (3, 4, 5) von oben gesehen jeweils eine Kontur (6) in U-Form und eine Dicke aufweisen, die vom Hinter- zum Vorderfuß abnimmt.

11. Vorrichtung nach Anspruch 10, **dadurch gekennzeichnet, dass** die Aussparung (17) derart positioniert ist, dass sie unter dem Absatz lokalisiert ist.

12. Vorrichtung nach Anspruch 10 oder Anspruch 11, **dadurch gekennzeichnet, dass** die reziproken Verschachtelungsmittel derart positioniert sind, dass sie unter dem Absatz lokalisiert sind.

13. Vorrichtung nach einem der Ansprüche 10 oder 12, **dadurch gekennzeichnet, dass** die elementaren Teile (3, 4, 5) eine Unterseite (8, 9, 10) aufweisen, die eine allgemeine untere Ebene definieren und eine Oberseite (11, 12, 13), die eine allgemeine obere Ebene definieren, die im Verhältnis zur allgemeinen unteren Ebene in einem Winkel zwischen 4 und 15° inklusive geneigt ist.

14. Vorrichtung nach einem der Ansprüche 10 bis 13, **dadurch gekennzeichnet, dass** der Keilabsatz (1) drei elementare Teile umfasst, nämlich ein ständiges Teil (5), das dazu bestimmt ist, mit dem Absatz im Kontakt zu sein, ein Zwischenteil (4), das dazu bestimmt ist, unter dem ständigen Teil (5) angebracht zu werden und ein Basisteil (3), das dazu bestimmt ist, unter dem Zwischenteil (4) angebracht zu werden, wobei sich der Keilabsatz (1) durch Entfernen der elementaren Teile (3, 4, 5) vom unteren Teilstück des Keilabsatzes (1) aus entwickelt.

15. Vorrichtung nach den Ansprüchen 13 und 14, **dadurch gekennzeichnet, dass**:
- das Basisteil (3) ein erstes zylindrisches Element (14) umfasst, das etwa senkrecht über die Oberseite (11) hervorsteht und eine Höhe aufweist, die kleiner ist als die Dicke des Zwischenteils (4) in dem Abschnitt des Zwischenteils (4), der dazu bestimmt ist, dem ersten Element (14) gegenüberzuliegen,
- das Zwischenteil (4) eine durchgehende Aussparung (17) aufweist mit einem Durchmesser, der etwa dem Durchmesser des ersten zylindrischen Elements (14) entspricht, wobei das erste zylindrische Element (14) dazu bestimmt ist, in die Aussparung (17) einzugreifen, und einen Kranz (18), der etwa senkrecht über der Oberseite (12) um die Aussparung (17) vorsteht, wobei der Innendurchmesser des Kranzes (18) etwa mit dem Durchmesser der Aussparung (17) identisch ist,
- das ständige Teil (5) eine Kranznut (21) umfasst, die von der Unterseite (10) eingebracht ist, wobei die Nut (21) einen Innendurchmesser und einen Außendurchmesser aufweist, die jeweils mit dem Innendurchmesser und dem Außendurchmesser des vorstehenden Kranzes (18) des Zwischenteils (4) etwa identisch sind, wobei die Nut (21) in ihrer Mitte ein zweites vorstehendes zylindrisches Element (22) definiert, dessen unteres Ende (23) sich etwa in der allgemeinen unteren Ebene des ständigen Teils (5) befindet, wobei die Nut (21) und das zweite zylindrische Element (22) dazu bestimmt sind, jeweils mit dem Kranz (18) und der Aussparung (17) zusammenzuarbeiten.
